# EUROPEAN PATENT SPECIFICATION

(11) **EP 2 231 112 B1**
(45) Date of publication and mention of the grant of the patent: **29.08.2012**
(21) Application number: 08700908.0
(22) Date of filing: 25.01.2008
(51) Int. Cl.: A61K 9/00

(54) **COMPRESSED CHEWING GUM COMPRISING STEROL**
KOMPRIMIERTES KAUGUMMI MIT EINEM STEROL
CHEWING-GUM COMPRIMÉ COMPRENANT DU STÉROL

(43) Date of publication of application: 29.09.2010
(73) Proprietor: Gumlink A/S, 7100 Vejle (DK)
(72) Inventor: STEENBERG, Lars Christian Kure, 7100 Vejle (DK)
(74) Representative: Mikkelsen, Hans Kristian Saaby
(86) International application number: PCT/DK2008/000037
(87) International publication number: WO 2009/092377

(56) References cited:
- WO-A-03/063605
- JP-A- 2006 211 909
- US-A1- 2002 110 531

## Description

### FIELD OF THE INVENTION

The present invention relates to the field of compressed chewing gum. In particular, the present invention provides a compressed chewing gum tablet comprising sterol compound.

### TECHNICAL BACKGROUND

It is widely recognized that sterols and derivatives thereof, e.g. stanols and stanol esters, can block cholesterol absorption sites in the human intestine thus helping to reduce cholesterol in humans.

These compounds are generally referred to hereinafter as sterol compounds. Too high cholesterol levels in humans may be the cause of atherosclerosis. This can lead to cardiovascular diseases such as myocardial infarction.

Sterols have similar chemical structures to cholesterol. Sources of sterols are plants and animals. Phytosterols, derived from plants, are considered to be part of a healthy diet. Many margarines, cereals and the like are enriched with sterols to offer the buyer the mentioned benefits.

Also new research points out anti-inflammatory effects of sterols with possible benefits for people suffering from auto-immune diseases and other inflammatory conditions in the body.

WO 03/063605 suggests the use of plant sterol in a conventional chewing gum composition. A method of manufacturing small sized particles of plant sterols for the use as additive in conventional chewing gum is disclosed.

There have been problems in prior art methods of manufacturing chewing gum to administer sterols to people in daily amounts large enough to have a positive cholesterol effect. Indeed indications have been seen that a daily intake of about 800 mg of sterols will be needed in order to have an effective amount. In order to administer this amount via a chewing gum, large amounts of sterols in each chewing gum will be necessary. A problem, however, is that release of sterols from the above-mentioned chewing gum tends to be ineffective.

US 7,258,851 discloses a chewing gum with a liquid centre filling containing stanol compounds.

The liquid centre filling may comprise enough stanol compounds to be effective with up to seven individual servings pr. day. However, the dispersion of the stanol compounds in the liquid for the liquid center fill requires further process steps such as heating, viscosity control, agitation, addition of further ingredients etc. rendering the process troublesome and expensive. Moreover the positioning of the stanol compounds inside the gum base will most likely cause that a substantial amount of the stanol compounds in the liquid center filling may be chewed into the gum base and thereby become trapped and ineffective.

It is therefore an objective of the present invention to provide an improved method for producing chewing gums containing effective amounts of sterols.

Moreover it is an objective of the present invention to provide a chewing gum containing an effective amount of sterols which are satisfyingly released.

### SUMMARY

The invention relates to a compressed chewing gum tablet comprising at least one chewing gum module, the chewing gum module including a chewing gum composition comprising chewing gum particles containing gum base,
wherein said chewing gum tablet comprises at least one sterol compound, and
wherein at least a part of said at least one sterol compound is located outside said chewing gum particles containing gum base.

Using compression technique allows incorporation of larger amounts of sterols in the chewing gum thereby facilitating enhancement of the sterol uptake in the body and thereby introducing effective means to reduce the cholesterol level in the blood of humans having elevated cholesterol levels.

It is surprising that by using the compression technique according to the provisions of the invention it is possible to add large amounts of sterols to a chewing gum and obtain a satisfying release of the sterols.

Indeed a chewing gum of standard size according to embodiments of the invention may have large amounts of above 200 mg sterol with effective release of between 50 and 95 % of the complete sterol content of the tablet and still be physically stable.

Phytosterols and phytostanols are associated with the fibres of fruits and vegetables and therefore not readily absorbed in the body from their natural sources. Compressed chewing gum tablets of the present invention are suitable carriers of concentrated amounts of sterols which may be more easily utilized by the human intestine system.

Moreover, release of sterols from the chewing gum may be obtained by keeping at least some of the sterols outside the gum base according to the provisions of the invention.

In an embodiment of the invention said at least one sterol compound is applied in powder form.

Sterols typically are commercially available as powders. These may without further processing be used as ingredient in compressed chewing gum formulation.

Powders are available which contribute very little to the taste of the chewing gum whereby excessive use of flavor to mask any unpleasant taste may be omitted. Using sterols directly as supplied in powdery form may enhance the stability of the sterols towards oxidation and other degradation processes when comprised in a chewing gum tablet thereby prolonging the shelf life of the chewing gum tablet when compared to other chewing gum formulations containing sterols.

In an embodiment of the invention said sterol compound comprises one or more substances selected from the group consisting of sterols, stanols, sterol esters, stanol esters and derivatives thereof.

In an embodiment of the invention at least a part of said at least one sterol compound is located in chewing gum particles free of gum base.

The chewing gum composition will typically comprise chewing gum particles, which are free of gum base. This may be particles of any chewing gum ingredient such as bulk sweetener, high-intensity sweetener, flavor, color, sterol compound etc., each of which may be alone or in combination in the chewing gum particles.

In an embodiment of the invention at least a part of said at least one sterol compound is located among the chewing gum particles free of gum base and the chewing gum particles containing gum base.

According to embodiments of the invention, the at least one sterol compound may be in chewing gum particles free of gum base, or possibly located between other chewing gum particles, both chewing gum particles containing gum base or chewing gum particles free of gum base.

In an embodiment of the invention said at least one sterol compound is contained in particles of which the at least one sterol compound make up more than 20, preferably more than 40, more preferably more than 60, even more preferably more than 80, and most preferably more than 90 % by weight of the particles.

By using particles comprising a high amount of sterol compounds, a high amount of sterol compound in the resulting tablet is made possible. Hereby it is further made possible and facilitated that a tablet with a very high amount of sterol compound can be obtained without necessarily turning the tablet larger than what is comfortable to the user.

In an embodiment of the invention said chewing gum particles containing gum base comprise further chewing gum ingredients.

According to an embodiment of the invention, chewing gum ingredients may comprise further components than the gum base components. Such further chewing gum ingredients may typically relate to sweetener, flavor, color etc. These ingredients are very often hydrophilic.

Furthermore, sterol compounds which may supplement a healthy diet may advantageously be administered to people by way of chewing gum. In particular sterol compounds are very well suited for incorporation in compressed chewing gum, especially when the amounts necessary for a desired effect are incorporable in compressed chewing gum of a desired size.

In an embodiment of the invention said chewing gum particles free of gum base comprise chewing gum ingredients.

In embodiments of the invention, the chewing gum particles may comprise chewing gum ingredients such as filler, coloring agent, flavoring agent, high-intensity sweetener, bulk sweetener, softener, emulsifier, acidulant, antioxidant, dry-binders, tabletting aids, anti-caking agents, enhancers, absorption enhancers, further conventional chewing gum ingredients and more.

In an embodiment of the invention said sterol compound is separately added to the chewing gum composition located outside the chewing gum particles containing gum base, comprised in chewing gum particles free of gum base and/or combinations thereof.

Placing sterol compound outside of the chewing gum particles containing gum base may facilitate fast and effective release of the sterol compound thereby maximizing the physiological effect of the sterol compound administered by way of the chewing gum tablet.

In an embodiment of the invention said chewing gum particles free of gum base comprise flavor.

In an embodiment of the invention, said chewing gum particles free of gum base comprises flavor in an amount of 0-60% by weight of said chewing gum tablet.

In an embodiment of the invention said chewing gum particles containing gum base comprise a part of said sterol compound.

Typically it will not be desired to have sterol compound in the chewing gum particles containing gum base, however in some embodiments it may be advantageous to have small part of the sterol compound located in the chewing gum particles containing gum base.

In an embodiment of the invention said compressed chewing gum tablet consists of one module.

In an embodiment of the invention said compressed chewing gum tablet comprises at least one gum base-free module.

In an embodiment of the invention said compressed chewing gum tablet comprises at least two modules.

In some embodiments of the invention three, four or more modules may be applied. Each of these may comprise gum base or be free of gum base, provided that at least one module comprises gum base.

In an embodiment of the invention said compressed chewing gum tablet comprises at least one gum base-free module and at least one gum base-containing module.

According to embodiments of the invention, the at least one sterol compound may be located in a gum base-free module, in a gum base-containing module or in both modules.

In an embodiment of the invention the gum base-free module constitute more than 50% by weight of the chewing gum tablet.

In some embodiments of the invention it is preferred that a gum base-free module is larger by weight than a gum base-containing module. This is especially the case when larger part of the sterol compound is positioned in the gum base-free module.

In an embodiment of the invention the ratio measured in % by weight of the whole tablet between the at least one gum base-containing module and the at least one gum base-free module is at least 65:35.

In some embodiments of the invention, the ratio between the layers may be up to 80:20 or even 90:10. However it will not always be advantageous that the gum base-free module is markedly smaller than the gum base-containing module.

In an embodiment of the invention said at least one gum base-free module comprises said at least one sterol compound.

Gum base comprising sterols will release those comparatively slowly. This may be beneficial for secondary functions of sterols such as anti-inflammatory action in the oral cavity.

An effective and fast release of sterols may be obtained by incorporating sterols in the gum base-free module. Since sterols may be an expensive ingredient in the chewing gum, it may be important to release as much as possible of the sterols from the chewing gum to ascertain an effective utilization of the sterols added.

In an embodiment of the invention said at least one gum base-containing module comprises said at least one sterol compound.

In order to increase the total amount of sterol in the chewing gum, in some embodiments of the invention some sterol may be positioned in the gum base-containing module in the form of powder together with the chewing gum granules of the module. As can be seen from the examples the release of sterol positioned between the chewing gum granules is good.

In an embodiment of the invention said at least one sterol compound is positioned in a module separate of said chewing gum particles containing gum base.

In an embodiment of the invention said at least one sterol compound and said chewing gum particles containing gum base are positioned in the same module.

In an embodiment of the invention the chewing gum particles free of gum base which comprises at least one sterol compound are compressible.

In an embodiment of the invention said at least one sterol compound is compressible.

In embodiments, where sterol compounds which are compressible are used, it is possible to use particles for compression which are constituted solely by the sterol compound. For sterol compound where this is possible, very large amounts of sterol compound may be positioned in the chewing gum tablet.

In an embodiment of the invention said at least one sterol compound is mixed with compressible materials prior to compression.

When using sterol compounds, which are not directly compressible, it may be advantageous according to embodiments of the invention to mix the sterol compound with another compressible material prior to compression. This may be e.g. a bulk sweetener such as xylitab.

In an embodiment of the invention said compressible materials are present in an amount of at least 20, at least 30, at least 40 or at least 50 % by weight of the relevant module.

For some sterol compounds which are not directly compressible, the presence of compressible material may in some embodiments of the invention be in relatively high amounts to ensure a compressible product.

In an embodiment of the invention at least a part of said chewing gum particles free of gum base consist of at least one sterol compound comprising substances selected from the group consisting of sterols, stanols, sterol esters, stanol esters and derivatives thereof.

Typically commercially available sterol compounds comprise a mixture of different sterols. Examples of these different sterols are β-Sitosterol, β-Sitostanol, Ergosterol, Campesterol, Campestanol, Stigmasterol, Brassicasterol and/or esters thereof.

In an embodiment of the invention said sterol compound comprises sterol esters and stanol esters in an amount of below 10% by weight of the sterol compound.

In general esters of sterols are often avoided in that they tend to be more lipophilic than sterols. Therefore a larger part of the sterols may be lost in the gum base if high amounts of sterol esters are applied.

In an embodiment of the invention said sterol compound is a mixture of at least one sterol and at least one stanol in a total amount of more than 30% by weight of the sterol compound.

Methods of extracting sterol compounds may result in compounds with high content of sterol and stanol relative to other ingredients. According to embodiments of the invention it may be possible to control the relative content of sterol and stanol in the sterol compound.

In an embodiment of the invention said sterol is selected from the group consisting of zoo-sterols and phyto-sterols.

In an embodiment of the invention said compressed chewing gum tablet comprises high intensity sweeteners in an amount of less than 1, preferably less than 0.5% by weight of the said compressed chewing gum tablet.

Reduction of high intensity sweetener content in embodiments of the present invention may provide lower production costs.

In an embodiment of the invention said compressed chewing gum tablet comprises high intensity sweeteners in an amount of less than 0.6% by weight of the said compressed chewing gum tablet and sterol compound in an amount of more than 100 mg.

In an embodiment of the invention said compressed chewing gum tablet comprises high intensity sweeteners in an amount of less than 0.6% by weight of said compressed chewing gum tablet, sterol compound in an amount of more than 50 mg, and bulk sweetener in an amount of more than 10% by weight of said compressed chewing gum tablet.

When applying very large amounts of sterol compound to the compressed chewing gum tablet there may be an increased risk that the chewing gum tablet will initially upon chewing disintegrate slightly more than a conventional compressed chewing gum tablet. However in the combination of large amount of sterol compound with a certain amount of bulk sweetener and high intensity sweetener, the disintegration will be masked by the initial pleasant taste from the sweeteners. Therefore it may actually be accepted that a slight disintegration, which may be inherent when applying very large amounts of sterol compounds, may occur.

In an embodiment of the invention said compressed chewing gum tablet comprises high intensity sweeteners in an amount of more than 0.05% by weight of the said compressed chewing gum tablet.

To adjust the taste of the chewing gum tablet comprising sterols it is often advantageous to add high intensity sweeteners. In some embodiments the high intensity sweetener may facilitate improved cohesion of the compressed chewing gum particles. Too high amounts of high intensity sweeteners may affect the taste of the chewing gum tablet unpleasantly.

In an embodiment of the invention the amount of high-intensity sweetener is less than the amount of sterol compounds in the chewing gum tablet.

In an embodiment of the invention said stanol is selected from the group of phytostanols.

In an embodiment of the invention said sterol is selected from the group of phytosterols.

Phytostanols and phytosterols are readily available from commercial sources and have a documented cholesterol effect.

In an embodiment of the invention said compressed chewing gum tablet comprises said at least one sterol compound in an amount of above 5 mg, preferably above 10 mg, most preferably above 20 mg.

In an embodiment of the invention said compressed chewing gum tablet comprises said at least one sterol compound in an amount of below 2000 mg, preferably below 1500 mg, most preferably below 1000 mg.

In an embodiment of the invention said compressed chewing gum tablet comprises said at least one sterol compound in an amount of between 50 and 500 mg.

It has been shown that a daily intake of about 800 mg of sterols may have the desired cholesterol effect. This daily intake can be achieved with few servings of chewing gum of the present invention thereby providing effective means to reduce the cholesterol level in the blood of humans having elevated cholesterol levels.

In an embodiment of the invention said compressed chewing gum tablet comprises bulk sweetener in an amount of at least 10%, preferably at least 15% and most preferably at least 20% by weight of the chewing gum tablet.

In embodiments of the present invention the amount of bulk sweetener in the compressed chewing gum tablet may be above 30, 40 or 50% by weight of the chewing gum tablet.

Substantial amounts of bulk sweetener may advantageously affect both taste and disintegration feel of compressed chewing gum tablets according to provisions of the invention.

In an embodiment of the invention said compressed chewing gum tablet comprises biodegradable polymers.

In an embodiment of the invention, said biodegradable gum base comprises at least one polymer selected from the group consisting of polyesters, poly(ester-carbonates), polycarbonates, polyester amides, polyhydroxy alkanoates, polypeptides, homopolymers of amino acids such as polylysine, proteins such as prolamin, and protein derivatives such as protein hydrolysates including a zein hydrolysate, or any combination thereof.

In an embodiment of the invention said compressed chewing gum tablet is coated.

A coating applied to the compressed chewing gum tablet may be a hard coating, a soft coating, a film coating, or a coating of any type that is known in the art, or a combination of such coatings.

In an embodiment of the invention said compressed chewing gum tablet has a volume of at least 0.15 cm³.

Larger volume chewing gum tablets may comprise sterols in amounts very relevant for lowering cholesterol levels in humans.

In an embodiment of the invention at least one module is compressed by a force of more than 13 kN.

Chewing gum tablets produced at a relatively high compression force may develop a certain desired brittleness and therefore disintegrate in a certain explosion-like manner upon chewing thereby freeing the sterols for utilization in the body before integration of substantial amounts of sterols in the gum base may occur.

In an embodiment of the invention said sterol compound is combined with at least one further active ingredient.

According to embodiments of the invention the sterol compound may be combined with one or more further active ingredients. Such active ingredients may be selected from the group consisting of pharmaceuticals, nutraceuticals, medicaments, nutrients, nutritional supplements, drugs, dental care agents, herbals, and the like and combinations thereof.

In further embodiments of the invention the active ingredient may be selected from the ATC anatomical groups consisting of agents acting on:
A alimentary tract and metabolism, B blood and blood forming organs, C cardiovascular system, D dermatologicals, G genito urinary system and sex hormones, H systemic hormonal preparations, J antiinfectives for systemic use, L antineoplastic and immunomodulating agents, M musculo-skeletal system, N nervous system, P antiparasitic products, insecticides and repellents, R respiratory system and S sensory organs, V various, or any combination thereof.

In certain embodiments of the invention active ingredients which may be combined with sterols is chosen e.g. among anti-plaque agents, anti-gingivitis agents, remineralization agents, whitening agents, fresh-breath agents, and anti-calculus agents.

Anti-plaque agents may include xylitol, maltitol, tricolsan, chlorhexidine, zinc acetate, zinc gluconate, zinc citrate, silver nitrate, copper, limonene, and pyridinium chloride.

Anti-gingivitis agents may include Coenzyme Q 10, lysozyme, papein, extracts of sassafras, chlorophyll, citral geraniol, cardamom, clove, cranberry, blueberry, Aronia melanocarpa, sage, carvacrol, eucalyptus, seaweed, magnolia bark, thyme, oregano, parsley, marjoram, cinnamon, lemon, lime, green tea, red tea, white tea, grapefruit and orange.

Whitening agents may include magnesium carbonate, calcium carbonate, calcium pyrophosphate, baking soda, sodium hexa-metaphosphate, magnesium silicate, silica, titanium dioxide, zinc oxide, and mixtures thereof.

Fresh-breath agents may include chlorohexidine, hexetidine, delmopinol, zinc oxide, zinc silicate, zinc carbonate, zinc acetate, zinc phosphate, zinc stannate, zinc citrate, zinc, zinc oxalate, zinc stearate, zinc chloride, zinc sulphate, zinc nitrate, zinc compounds as a complexes, green tea, red tea, white tea, black tea, thyme, eucalyptus, and mixtures thereof.

Moreover the invention relates to a compressed chewing gum tablet comprising at least one chewing gum module comprising a chewing gum composition, wherein the chewing gum composition comprises chewing gum particles containing gum base and chewing gum particles free of gum base,
wherein said chewing gum tablet comprises at least one sterol compound, and
wherein at least a part of said chewing gum particles free of gum base comprise at least a part of said at least one active ingredient.

Moreover the invention relates to a method for the production of a chewing gum according to any of the claims 1 16 wherein said method comprises at least one compression step.

In an embodiment of the invention said method comprises the addition of said sterols, stanols, sterol esters, stanol esters and derivatives thereof as powder.

In an embodiment of the invention said method comprises a way of administering effective amounts of said sterols, stanols and/or esters thereof to people who need to lower their cholesterol level in the blood.

It is known that sterol compounds may have anti-inflammatory effects and thereby helping to counteract inflammatory conditions. An advantageous method of administering sterol compounds to human beings is through chewing gum, in that the chewing of a chewing gum may facilitate a combined effect of lowering high cholesterol levels and treating of inflammatory conditions from each piece of chewing gum. Inflammatory conditions may e.g. be in the mouth cavity.

### DRAWINGS

The invention will be described with reference to the drawings of which
fig. 1a -1b illustrate a two-layer compressed tablet according to an embodiment of the invention,
fig. 2a -2b illustrate a three layer compressed tablet according to an embodiment of the invention,
fig. 2c -2d illustrate a four layer compressed tablet according to an embodiment of the invention,
fig. 3a-3b illustrate a further two layer compressed tablet according to an embodiment of the invention,
fig. 4a-4b illustrate a further two layer compressed tablet according to an embodiment of the invention, and where
fig. 5a-5b illustrate a further two layer compressed tablet according to an embodiment of the invention.

### DETAILED DESCRIPTION

With the present invention, as described in the following, compressed chewing gum tablets comprising at least one sterol compound comprising substances from the group comprising sterols, stanols, sterol esters, stanol esters and derivatives thereof.

An advantageous release of sterol compounds is obtained by the incorporation of sterols in compressed chewing gum tablets comprising one or more modules, of which at least one module comprises sterols, meaning throughout this document sterols and derivatives thereof like stanol, sterol esters, stanol esters and/or combinations thereof.

The word particle throughout this document should be understood as broadly as possibly being powder, granules, agglomerates or the like. In the same manner the word granule may be understood as a particle and generally the four terms may be used interchangeably. Particles and thereby granules may typically be compressible at least to the extent that it is possible to compress the tablet in question.

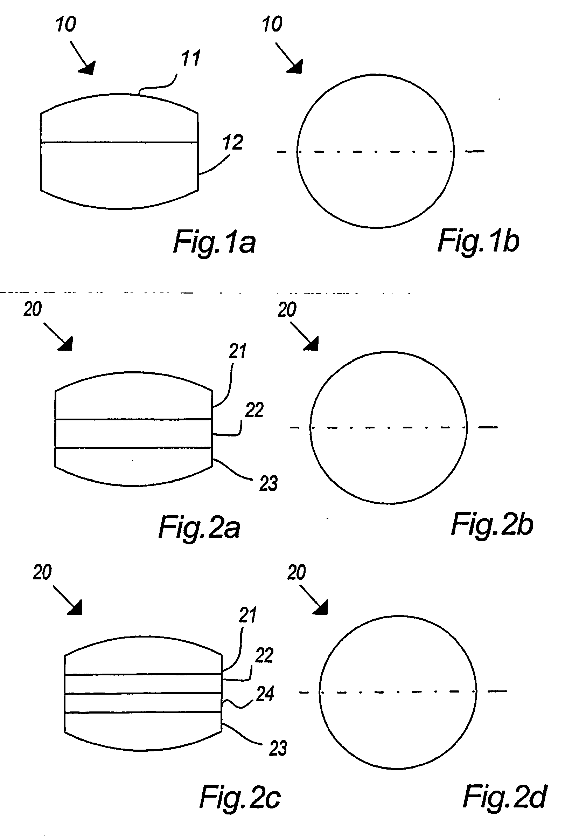

Fig. 1a illustrates a cross-section of a compressed multi modular chewing gum tablet according to the invention and illustrated in fig. 1b.

According to the illustrated embodiment, each module is simply comprised by a layer.

The illustrated chewing gum tablet 10 may for example weigh approximately 2.0 gram and comprise a first GB-containing chewing gum module 12 and a second GB-free module 10 (GB: gum base).

The illustrated tablet has an approximate diameter of 16 mm and a thickness at the thickest point in the center of approximately 7 mm.

The two modules 11 and 12 are adhered to each other. Different processes may be applied for the purpose. However, according to a preferred embodiment of the invention, the mutual adhering between the two layers is obtained by the compression of one module 11 onto the other 12.

According to an embodiment of the invention, the illustrated chewing gum tablet 10 may be provided with a coating, e.g. a film coating.

It should be noted that various concentrations of gum base in the different modules (here: layers) may be applied within the scope of the invention. For example both modules 11 and 12 shown in fig. 1a may comprise gum base.

The modules may for instance comprise compressible chewing gum ingredients, for example sweeteners and flavors, more or less pre-processed for the purpose of facilitating a true compression. Other ingredients to be emphasized here are sterols.

In other applications, e.g. for the purpose of establishing different release profiles the different modules may comprise different content of gum base.

The tablet may moreover comprise (not shown) one or several barrier layers adapted for establishment of a barrier between inter-reacting ingredients, such as certain acids and flavors.

Fig. 2a illustrates a cross-section of a compressed multi modular chewing gum tablet according to an embodiment of the invention and illustrated in fig. 2b from above.

The illustrated embodiment 20 comprises a three-module chewing gum of which the each of the layers 21, 22, and 23 optionally comprise a gum base incorporated chewing gum module having a certain gum base concentration. As an example module 22 may contain gum base and modules 21 and 23 may be substantially gum base-free.

Modules without gum base, which could be chewing gum module 21 and 23, may for example comprise compressed chewing gum ingredients, such as sweeteners, flavor, freeze-dried fruit, sterols, etc.

Modules not containing gum base, here modules 21 and 23, may ensure a fast release of sterol compound and avoid too much of the sterol compound to be trapped inside the gum base. Furthermore it is possible with the modules not containing gum base to add an amount of bulk sweetener or other chewing gum ingredients, which are quickly released when the tablet is chewed.

Fig. 2c and 2d illustrate a cross-section of a compressed multi modular chewing gum tablet according to an embodiment of the invention with the same possibilities as explained above with reference to figs. 2a and 2b.

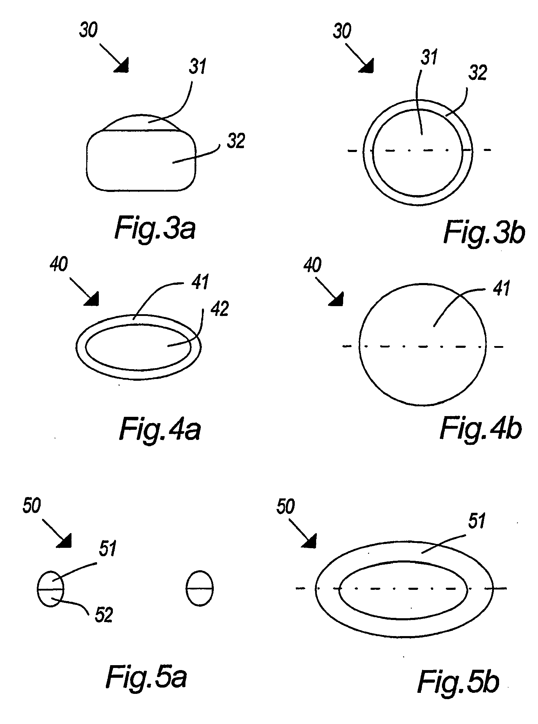

Fig. 3a illustrates a cross-section of a compressed multi modular chewing gum tablet 30 according to the invention and illustrated in fig. 3b from above.

The illustrated chewing gum tablet 30 comprises a gum base incorporated chewing gum module 32 upon which another gum base-free chewing gum module 31 is arranged.

Fig. 4a illustrates a cross-section of a further compressed multi-modular chewing gum tablet 40 according to an embodiment of the invention and illustrated in fig. 4b from above.

The tablet 40 differs somewhat from the other described tablets in the sense that the tablet comprises a compressed GB-incorporated chewing gum module 42 forming a gum center. The module 42 is encapsulated by a surrounding module 41.

Fig. 5a illustrates a cross-section of a compressed multi-modular chewing gum tablet 50 according to an embodiment of the invention and illustrated in fig. 5b from above.

According to the illustrated embodiment, showing a ring-formed two layer tablet 50, a base chewing gum module 52 comprises a certain concentration of gum base, whereas the other module 51 may be essentially gum base-free.

The formulations, examples of compositions and layers given herein are exemplary and only given for the purpose of evaluating and explaining different features of the invention. Compositions and the combinations of layers may be varied significantly within the scope of the invention. Specific variations and details with respect to ingredients, formulations and compositions within the scope of the invention are given below.

In accordance with the general principles in manufacturing a chewing gum tablet within the scope of the invention, variations of different suitable ingredients are listed and explained below.

Chewing gum of the present invention typically comprises a water-soluble portion, a water-insoluble chewable gum base portion and flavoring agents. The water-soluble portion dissipates with a portion of the flavoring agent over a period of time during chewing. The gum base portion is retained in the mouth throughout the chew. The term chewing gum refers to both a chewing and bubble type gum in its general sense.

The gum base is the masticatory substance of the chewing gum, which imparts the chew characteristics to the final product.

The insoluble portion of the gum typically may contain any combination of elastomers, vinyl polymers, elastomer plasticizers, waxes, softeners, fillers and other optional ingredients such as colorants and antioxidants.

The composition of gum base formulations can vary substantially depending on the particular product to be prepared and on the desired masticatory and other sensory characteristics of the final product. However, typical ranges (% by weight) of the above gum base components are: 5 to 80% by weight elastomeric compounds, 5 to 80% by weight elastomer plasticizers, 0 to 40% by weight of waxes, 5 to 35% by weight softener, 0 to 50% by weight filler, and 0 to 5% by weight of miscellaneous ingredients such as antioxidants, colorants, etc. The gum base may comprise about 5 to about 95 percent, by weight, of the chewing gum, more commonly the gum base comprises 10 to about 60 percent, by weight, of the gum.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in base. This may be important when one wants to provide more elastomeric chain exposure to the alkane chains of the waxes.

The elastomer compounds may be of natural origin but are preferably of synthetic origin, preferably synthetic polyesters.

It is noted that gum base or gum granules may also include components typically referred to as chewing gum ingredients.

The chewing gum may, according to embodiments of the invention, comprise conventionally non-biodegradable polymers, such as natural resins, synthetic resins and/or synthetic or natural elastomers.

According to an embodiment of the invention, at least a part of the polymers of the chewing gum are biodegradable.

In an embodiment of the invention, the chewing gum may comprise combinations of biodegradable polymers and polymers generally regarded as non-biodegradable, such as natural resins, synthetic resins and/or synthetic/natural elastomers.

In an embodiment of the invention, said natural resin comprises terpene resins, e.g. derived from alpha-pinene, beta-pinene, and/or d-limonene, natural terpene resins, glycerol esters of gum rosins, tall oil rosins, wood rosins or other derivatives thereof such as glycerol esters of partially hydrogenated rosins, glycerol esters of polymerized rosins, glycerol esters of partially dimerised rosins, pentaerythritol esters of partially hydrogenated rosins, methyl esters of rosins, partially hydrogenated methyl esters of rosins or pentaerythritol esters of rosins and combinations thereof.

Examples of generally non-biodegradable synthetic resins include polyvinyl acetate, vinyl acetate-vinyl laurate copolymers and mixtures thereof. Examples of non-biodegradable synthetic elastomers include, but are not limited to, synthetic elastomers listed in Food and Drug Administration, CFR, Title 21, Section 172,615, the Masticatory Substances, Synthetic) such as polyisobutylene. e.g. having a gel permeation chromatography (GPC) average molecular weight in the range of about 10,000 to 1,000,000 including the range of 50,000 to 80,000, isobutylene-isoprene copolymer (butyl elastomer), styrene-butadiene copolymers e.g. having styrene-butadiene ratios of about 1:3 to 3:1, polyvinyl acetate (PVA), e.g. having a GPC average molecular weight in the range of 2,000 to 90,000 such as the range of 3,000 to 80,000 including the range of 30,000 to 50,000, where the higher molecular weight polyvinyl acetates are typically used in bubble gum base, polyisoprene, polyethylene, vinyl acetate-vinyl laurate copolymer e.g. having a vinyl laurate content of about 5 to 50% by weight such as 10 to 45% by weight of the copolymer, and combinations hereof.

The elastomers (rubbers) employed in the gum base may vary depending upon various factors such as the type of gum base desired, the texture of gum composition desired and the other components used in the composition to make the final chewing gum product. The elastomer may be any water-insoluble polymer known in the art, and includes those gum polymers utilized for chewing gums and bubble gums. Illustrative examples of suitable polymers in gum bases include both natural and synthetic elastomers. For example, those polymers which are suitable in gum base compositions include, without limitation, natural substances (of vegetable origin) such as chicle gum, natural rubber, crown gum, nispero, rosidinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate and the like, and mixtures thereof.

It is common in the industry to combine in a gum base a synthetic elastomer having a high molecular weight and a synthetic elastomer having a low molecular weight. Examples of such combinations are polyisobutylene and styrene-butadiene, polyisobutylene and polyisoprene, polyisobutylene and isobutylene-isoprene copolymer (butyl rubber) and a combination of polyisobutylene, styrene-butadiene copolymer and isobutylene isoprene copolymer, and all of the above individual synthetic polymers in admixture with polyvinyl acetate, vinyl acetate-vinyl laurate copolymers, respectively and mixtures thereof.

Examples of natural resins are: Natural rosin esters, often referred to as ester gums including as examples glycerol esters of partially hydrogenated rosins, glycerol esters of polymerised rosins, glycerol esters of partially dimerized rosins, glycerol esters of tally oil rosins, pentaerythritol esters of partially hydrogenated rosins, methyl esters of rosins, partially hydrogenated methyl esters of rosins, pentaerythritol esters of rosins, synthetic resins such as terpene resins derived from alpha-pinene, beta-pinene, and/or d-limonene, and natural terpene resins.

The chewing gum according to embodiments of the invention may be provided with an outer coating.

The applicable hard coating may be selected from the group comprising of sugar coating and a sugarless coating and a combination thereof. The hard coating may e.g. comprise 50 to 100% by weight of a polyol selected from the group consisting of sorbitol, maltitol, mannitol, xylitol, erythritol, lactitol and Isomalt and variations thereof. In an embodiment of the invention, the outer coating is an edible film comprising at least one component selected from the group consisting of an edible film-forming agent and a wax. The film-forming agent may e.g. be selected from the group comprising cellulose derivative, a modified starch, a dextrin, gelatine, shellac, gum arabic, zein, a vegetable gum, a synthetic polymer and any combination thereof. In an embodiment of the invention, the outer coating comprises at least one additive component selected from the group comprising of a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, an acid and an agent capable of accelerating the after-chewing degradation of the degradable polymer.

In the manufacturing of conventional chewing gum, the ingredients, generally, may be mixed by first melting the gum base and adding it to the running mixer. Colors, active agents and/or emulsifiers may also be added at this time. A softener such as glycerin may also be added at this time, along with syrup and a portion of the bulking agent/sweetener. Further portions of the bulking agent/sweetener may then be added to the mixer. A flavoring agent is typically added with the final portion of the bulking agent/sweetener. A high-intensity sweetener is preferably added after the final portion of bulking agent and flavor has been added.

The entire mixing procedure typically takes from five to fifteen minutes, but longer mixing times may sometimes be required. Those skilled in the art will recognize that many variations of the above-described procedure may be followed. Including the one-step method described in US patent application 2004/0115305 hereby incorporated as reference. Chewing gums are formed by extrusion, compression, rolling and may be centre filled with liquids and/or solids in any form.

When manufacturing a compressed chewing gum tablet another method is applied, which is basically very different from the above described, but may broadly be described as an initial conventional mixing of the gum base, as above described, followed by a granulation of the obtained gum base mix. The obtained chewing gum granules may then be mixed with further chewing gum ingredients, such as sweeteners and flavor. This final granule mix may then be compressed under high pressure (typically when applying cooling) into a chewing gum tablet. For each compression a layer is made and in this way it is possible to make multi-layered chewing gums, such as two, three or four layers, wherein each layer may include an individual composition, i.e. different active ingredients may be used for medical purposes or different colors may be used for visual purposes, etc.

In further embodiments of the present invention, a chewing gum may also be provided with an outer coating, which may be a hard coating, a soft coating, a film coating, or a coating of any type that is known in the art, or a combination of such coatings. The coating may typically constitute 0.1 to 75% by weight of a coated chewing gum piece.

One preferred outer coating type is a hard coating, which term is including sugar coatings and sugar-free (or sugarless) coatings and combinations thereof. The object of hard coating is to obtain a sweet, crunchy layer, which is appreciated by the consumer and to protect the gum centers. In a typical process of providing the chewing gum centers with a protective sugar coating the gum centers are successively treated in suitable coating equipment with aqueous solutions of crystallizable sugar such as sucrose or dextrose, which, depending on the stage of coating reached, may contain other functional ingredients, e.g. fillers, colors, etc.

In one presently preferred embodiment, the coating agent applied in a hard coating process is a sugarless coating agent, e.g. a polyol including as examples sorbitol, maltitol, mannitol, xylitol, erythritol, lactitol and isomalt or e.g. a mono- di-saccharide including as example trehalose.

Or alternatively a sugar-free soft coating e.g. comprising alternately applying to the centers a syrup of a polyol or a mono- di-saccharide, including as examples sorbitol, maltitol, mannitol, xylitol, erythritol, lactitol, isomalt and trehalose.

In further useful embodiments, a film coating is provided by film-forming agents such as a cellulose derivative, a modified starch, a dextrin, gelatine, zein, shellec, gum arabic, a vegetable gum, a synthetic polymer, etc. or a combination thereof.

In an embodiment of the invention, the outer coating comprises at least one additive component selected from the group comprising a binding agent, a moisture-absorbing component, a film-forming agent, a dispersing agent, an antisticking component, a bulking agent, a flavoring agent, a coloring agent, a pharmaceutically or cosmetically active component, a lipid component, a wax component, a sugar, and an acid.

A coated chewing gum center according to embodiments of the invention may have any form, shape or dimension that permits the chewing gum center to be coated using any conventional coating process.

It should however be noted that application of different coating should be done with care as compressed chewing gum tablets may be negatively affected by direct contact with moisture or water.

The composition of gum base formulations can vary substantially depending on the particular product to be prepared and on the desired masticatory and other sensory characteristics of the final product. However, typical ranges of the above gum base components are: 5 to 80% by weight of elastomeric compounds, 5 to 80% by weight of elastomer plasticizers, 0 to 40% by weight of waxes, 5 to 35% by weight of softener, 0 to 50% by weight of filler, and 0 to 5% by weight of miscellaneous ingredients such as antioxidants, colorants, etc. The gum base may comprise about 5 to about 95% by weight of the chewing gum, more commonly; the gum base comprises 10 to about 60% by weight of the gum.

Elastomers provide the rubbery, cohesive nature to the gum, which varies depending on this ingredient's chemical structure and how it may be compounded with other ingredients. Elastomers suitable for use in the gum base and gum of the present invention may include natural or synthetic types.

Elastomer plasticizers vary the firmness of the gum base. Their specificity on elastomer inter-molecular chain breaking (plasticizing) along with their varying softening points cause varying degrees of finished gum firmness and compatibility when used in gum base. This may be important when one wants to provide more elastomeric chain exposure to the alkanic chains of the waxes.

If desired, conventional elastomers or resins may be supplemented or substituted by biodegradable polymers.

Biodegradable polymers that may be used in the chewing gum of the present invention may be homopolymers, copolymers or terpolymers, including graft- and block-polymers.

Useful biodegradable polymers, which may be applied as gum base polymers in the chewing gum of the present invention, may generally be prepared by step-growth polymerization of di-, tri- or higher-functional alcohols or esters thereof with di-, tri- or higher-functional aliphatic or aromatic carboxylic acids or esters thereof. Likewise, also hydroxy acids or anhydrides and halides of polyfunctional carboxylic acids may be used as monomers. The polymerization may involve direct polyesterification or transesterification and may be catalyzed.

The usually preferred polyfunctional alcohols contain 2 to 100 carbon atoms as for instance polyglycols and polyglycerols.

Gum base polymers may both be resinous and elastomeric polymers.

Suitable biodegradable gum base polymers include polyesters, polycarbonates, polyesteramides, polyesterurethanes, polyamides, prolamine, and combinations thereof.

The chewing gum may include any component known in the chewing gum art. For example, the chewing gum may include elastomers, bulking agents, waxes, elastomer solvents, emulsifiers, plasticizers, fillers, and mixtures thereof.

The chewing gum according to embodiments of the invention may comprise coloring agents. According to an embodiment of the invention, the chewing gum may comprise color agents and whiteners such as FD&C-type dyes and lakes, fruit and vegetable extracts, titanium dioxide and combinations thereof.

Further useful chewing gum base components include antioxidants, e.g. butylated hydroxytoluene (BHT), butyl hydroxyanisol (BHA), propylgallate and tocopherols, and preservatives.

A gum base formulation may, in accordance with the present invention, comprise one or more softening agents e.g. sucrose esters including those disclosed in WO 00/25598, which is incorporated herein by reference, tallow, hydrogenated tallow, hydrogenated and partially hydrogenated vegetable oils, cocoa butter, degreased cocoa powder, glycerol monostearate, glyceryl triacetate, lecithin, mono-, di- and triglycerides, acetylated monoglycerides, lanolin, sodium stearate, potassium stearate, glyceryl lecithin, propylene glycol monostearate, glycerine, fatty acids (e.g. stearic, palmitic, oleic and linoleic acids) and combinations thereof. As used herein the term "softener" designates an ingredient, which softens the gum base or chewing gum formulation and encompasses waxes, fats, oils, emulsifiers, surfactants and solubilisers.

To soften the gum base further and to provide it with water-binding properties, which confer to the gum base a pleasant smooth surface and reduce its adhesive properties, one or more emulsifiers is/are usually added to the composition, typically in an amount of 0 to 18% by weight, preferably 0 to 12% by weight of the gum base. Useful emulsifiers can include, but are not limited to, glyceryl monostearate, propylene glycol monostearate, mono- and diglycerides of edible fatty acids, lactic acid esters and acetic acid esters of mono- and diglycerides of edible fatty acids, acetylated mono and diglycerides, sugar esters of edible fatty acids, Na-, K-, Mg- and Ca-stearates, lecithin, hydroxylated lecithin and the like and mixtures thereof are examples of conventionally used emulsifiers which can be added to the chewing gum base. In case of the presence of a biologically or pharmaceutically active ingredient as defined below, the formulation may comprise certain specific emulsifiers and/or solubilisers in order to disperse and release the active ingredient.

Waxes and fats are conventionally used for the adjustment of the texture and for softening of the chewing gum base when preparing chewing gum bases. In connection with the present invention, any conventionally used and suitable type of natural and synthetic wax and fat may be used, such as for instance rice bran wax, polyethylene wax, petroleum wax (refined paraffin and microcrystalline wax), sorbitan monostearate, tallow, propylene glycol, paraffin, beeswax, carnauba wax, candelilla wax, cocoa butter, degreased cocoa powder and any suitable oil or fat, as e.g. completely or partially hydrogenated vegetable oils or completely or partially hydrogenated animal fats.

A chewing gum base formulation may, if desired, include one or more fillers/texturisers including as examples, magnesium and calcium carbonate, sodium sulphate, ground limestone, silicate compounds such as magnesium and aluminum silicate, kaolin and clay, aluminum oxide, silicium oxide, talc, titanium oxide, mono-, di- and tri-calcium phosphates, cellulose polymers, such as wood, and combinations thereof.

In addition to a water insoluble gum base portion, a typical chewing gum includes a water soluble bulk portion and one or more flavoring agents. The water-soluble portion may include bulk sweeteners, high-intensity sweeteners, flavoring agents, softeners, emulsifiers, colors, acidulants, buffering agents, fillers, antioxidants, and other components that provide desired attributes.

Combinations of sugar and/or non-sugar sweeteners can be used in the chewing gum formulation processed in accordance with the invention. Additionally, the softener may also provide additional sweetness such as aqueous sugar or alditol solutions.

Useful sugar sweeteners are saccharide-containing components commonly known in the chewing gum art including, but not limited to, sucrose, dextrose, maltose, dextrins, trehalose, D-tagatose, dried invert sugar, fructose, levulose, galactose, corn syrup solids, and the like, alone or in combination.

Sorbitol can be used as a non-sugar sweetener. Other useful non-sugar sweeteners include, but are not limited to, other sugar alcohols such as mannitol, xylitol, hydrogenated starch hydrolysates, maltitol, isomaltol, erythritol, lactitol and the like, alone or in combination.

High-intensity artificial sweetening agents can also be used alone or in combination with the above sweeteners. Preferred high-intensity sweeteners include, but are not limited to sucralose, aspartame, salts of acesulfame, alitame, neotame, twinsweet, saccharin and its salts, cyclamic acid and its salts, glycyrrhizin, dihydrochalcones, thaumatin, monellin, stevioside and the like, alone or in combination. In order to provide longer lasting sweetness and flavor perception, it may be desirable to encapsulate or otherwise control the release of at least a portion of the artificial sweetener. Techniques such as wet granulation, wax granulation, spray drying, spray chilling, fluid bed coating, coascervation, encapsulation in yeast cells and fiber extrusion may be used to achieve the desired release characteristics. Encapsulation of sweetening agents can also be provided using another chewing gum component such as a resinous compound.

Usage level of the high-intensity artificial sweetener will vary considerably and will depend on factors such as potency of the sweetener, rate of release, desired sweetness of the product, level and type of flavor used and cost considerations. Thus, the active level of high-potency artificial sweetener may vary from about 0 to about 8% by weight, preferably 0.001 to about 5% by weight.

If a low-calorie gum is desired, a low-caloric bulking agent can be used. Examples of low caloric bulking agents include polydextrose, Raftilose, Raftilin, fructooligosaccharides (NutraFlora^{®}), palatinose oligosaccharides; guar gum hydrolysates (e.g. Sun Fiber^{®}) or indigestible dextrins (e.g. Fibersol^{®}). However, other low-calorie bulking agents can be used.

The chewing gum according to the present invention may contain aroma agents and flavoring agents including natural and synthetic flavorings e.g. in the form of natural vegetable components, essential oils, essences, extracts, powders, including acids and other substances capable of affecting the taste profile. Examples of liquid and powdered flavorings include coconut, coffee, chocolate, vanilla, grape fruit, orange, lime, menthol, liquorice, caramel aroma, honey aroma, peanut, walnut, cashew, hazelnut, almonds, pineapple, strawberry, raspberry, tropical fruits, cherries, cinnamon, peppermint, wintergreen, spearmint, eucalyptus, and mint, fruit essence such as from apple, pear, peach, strawberry, apricot, raspberry, cherry, pineapple, and plum essence. The essential oils include peppermint, spearmint, menthol, eucalyptus, clove oil, bay oil, anise, thyme, cedar leaf oil, nutmeg, and oils of the fruits mentioned above.

The chewing gum flavor may be a natural flavoring agent, which is freeze-dried, preferably in the form of a powder, slices or pieces or combinations thereof. The particle size may be less than 3 mm, less than 2 mm or more preferred less than 1 mm, calculated as the longest dimension of the particle. The natural flavoring agent may be in a form where the particle size is from about 3 µm to 2 mm, such as from 4 µm to 1 mm. Preferred natural flavoring agents include seeds from fruit e.g. from strawberry, blackberry and raspberry.

Various synthetic flavors, such as mixed fruit flavors may also be used in the present chewing gum centers. As indicated above, the aroma agent may be used in quantities smaller than those conventionally used. The aroma agents and/or flavors may be used in the amount from 0.01 to about 30% by weight of the final product depending on the desired intensity of the aroma and/or flavor used. Preferably, the content of aroma/flavor is in the range of 0.2 to 5%, more preferably 0.5 to 3%, by weight of the total composition.

In an embodiment of the invention, the flavoring agents comprise natural and synthetic flavorings in the form of natural vegetable components, essential oils, essences, extracts, powders, including acids and other substances capable of affecting the taste profile.

In one embodiment of the invention, the flavor may be used as taste masking in chewing gum comprising active ingredients, which by themselves have undesired taste or which alter the taste of the formulation.

Further chewing gum ingredients, which may be included in the chewing gum according to the present invention, include surfactants and/or solubilisers, especially when pharmaceutically or biologically active ingredients are present. As examples of types of surfactants to be used as solubilisers in a chewing gum composition according to embodiments of the invention, reference is made to H.P. Fiedler, Lexikon der Hilfstoffe für Pharmacie, Kosmetik und Angrenzende Gebiete, pages 63-64 (1981) and the lists of approved food emulsifiers of the individual countries. Anionic, cationic, amphoteric or non-ionic solubilisers can be used. Suitable solubilisers include lecithin, polyoxyethylene stearate, polyoxyethylene sorbitan fatty acid esters, fatty acid salts, mono and diacetyl tartaric acid esters of mono and diglycerides of edible fatty acids, citric acid esters of mono and diglycerides of edible fatty acids, saccharose esters of fatty acids, polyglycerol esters of fatty acids, polyglycerol esters of interesterified castor oil acid (E476), sodium stearoyllatylate, sodium lauryl sulfate and sorbitan esters of fatty acids and polyoxyethylated hydrogenated castor oil (e.g. the product sold under the trade name CREMOPHOR), block copolymers of ethylene oxide and propylene oxide (e.g. products sold under trade names PLURONIC and POLOXAMER), polyoxyethylene fatty alcohol ethers, polyoxyethylene sorbitan fatty acid esters, sorbitan esters of fatty acids and polyoxyethylene steraric acid esters.

Particularly suitable solubilisers are polyoxyethylene stearates, such as for instance polyoxyethylene(8)stearate and polyoxyethylene(40)stearate, the polyoxyethylene sorbitan fatty acid esters sold under the trade name TWEEN, for instance TWEEN 20 (monolaurate), TWEEN 80 (monooleate), TWEEN 40 (monopalmitate), TWEEN 60 (monostearate) or TWEEN 65 (tristearate), mono and diacetyl tartaric acid esters of mono and diglycerides of edible fatty acids, citric acid esters of mono and diglycerides of edible fatty acids, sodium stearoyllatylate, sodium laurylsulfate, polyoxyethylated hydrogenated castor oil, blockcopolymers of ethylene oxide and propyleneoxide and polyoxyethylene fatty alcohol ether. The solubiliser may either be a single compound or a combination of several compounds.

Sterol compounds may advantageously be applied in a compressed chewing gum according to the invention, of which the preferred sterol compounds to be used are at least one substance selected from the group of sterols, stanols, sterol esters, stanol esters and derivatives thereof.

Antioxidants can include materials that scavenge free radicals. In some embodiments, antioxidants can include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some embodiments, exempt from certification or natural colors can include, but are not limited to annatto extract, (E 160b), bixin, norbixin, astaxanthin, dehydrated beets (beet powder), beetroot red/betanin (E 162), ultramarine blue, canthaxanthin (E161g), cryptoxanthin (E 161 c), rubixanthin (E161d), violanxanthin (E161e), rhodoxanthin (E161f), caramel (E150(a-d)), β-apo-8'-carotenal (E160e), β-carotene (E160a), alpha carotene, gamma carotene, ethyl ester of beta-apo-8 carotenal (E160f), fiavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminum (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These consist of the aluminum salts of FD&C dyes extended on an insoluble substrate of alumina hydrate. Additionally, in some embodiments, certified colors can be included as calcium salts.

The sterol compound may be applied in a gum base-containing module and/or a gum base-free module depending on the desired release profile.

In the present context, the terms granule and particle are used interchangeable in the sense that a granule or particle for use in a compression process is regarded to be a relatively small object, which together with other granules or particles may be compressed into a stable chewing gum tablet. The granules or particles may be produced in several different ways. A gum base-containing granule of particle may typically be produced substantially into the desired shape by means of an extrusion process or alternatively be produced on the basis of a gum base-containing mass which is subsequently separated into particles of a smaller size.

The following non-limiting examples illustrate different variations of compressed chewing gum tablets according to embodiments of the invention. The examples are meant for indicating the inventive concept; hence the mentioned examples should not be understood as exhaustive for the present invention.

### Example 1

### Preparation of gum base

The applied gum base used had the following composition and was mixed in a conventional mixing process:

| | |
|---|---|
| elastomer: | 19% by weight |
| natural resin: | 20% by weight |
| synthetic resin: | 20% by weight |
| fat/fillers: | 26% by weight |
| wax: | 15% by weight |

Obviously within the scope of the invention gum base may be prepared by other processes such as a one-step process or any other conventional process.

### Example 2

### Preparation of chewing gum granules

The gum base of example 1 was used in the manufacture of chewing gum products according to embodiments of the invention.

An extruder (Leistritz ZSE/BL 360 kw 104, available from Leistritz GmbH, Germany) extruded the composition through the die plate into the liquid filled chamber (granulator A5 PAC 6, available from GALA GmbH, Germany). Descriptions of the extruder and the granulator may be found in e.g. WO 2004/098305 .

Gum base in the form of pellets and menthol flavor crystals (MENTHOL BPIUSP, available from SHARP MENTHOL INDIA LIMITED, India), were mixed in the extruder. The chewing gum composition had the composition as shown in table 1.

**Table 1.**

| **Ingredient** | **Amount (wt%)** |
|---|---|
| gum base | 97 |
| menthol flavor crystals | 3 |

The extruder delivered the composition at a feed rate of 400 kg/h to the die plate. The extruder screw speed was 247 rpm. The minimum temperature in the extruder was 44°C and a temperature of less than 70°C was maintained along about % of the extruder barrel length, until the composition passed the heating device in the outlet end of the extruder. Here the composition was heated to an extruder exit temperature of 109°C. The extruder and the granulator produced a pressure difference of 71 bar.

The composition was extruded through the die plate, which was heated to a temperature of 177°C and had 696 holes with a diameter of 0.36mm. In the granulator chamber the extruded composition was cut to granules by a cutter with 8 blades and cutter speed 1999 rpm. The particles were cooled and transported to the strainer unit (a centrifugal dryer TWS 20, available from GALA GmbH, Germany) in water with temperature 11°C and flow 22 m³/h. The average cooling and transport time in water was approx. 60 seconds. The particle rate was 400 kg/h and the average diameter of the obtained particles was 0.93mm.

The cooling and transport stage carried out in water in this example could be carried out in other media as well such as e.g. air.

Finally, talc was attached to the granules in between de-watering and conveyance to the tablet pressing apparatus or storing or packaging e.g. for transportation.

It should be emphasized that the above-applied manufacturing methods of gum base-containing granules are only exemplary and although advantageous not mandatory. Thus, an alternative method for the manufacturing of gum base-containing granules may be a conventional process involving that an initial gum base-containing mass is cooled to a low temperature, preferably to a temperature below 0°C and then mechanically particulated into small relatively irregular gum base-containing granules. If desired, these particles may be sieved or further processed to obtain a homogeneous granule blend. An example of such alternative process is disclosed in WO 2004/073691 as applied in a multi-layer tablet, hereby incorporated by reference.

### Example 3

### Preparation of sterol containing single-layer tablets

The composition of particles from Example 2 was compressed into a tablet according to the following process:
High-intensity sweeteners: aspartame powder and acesulfame K; bulk sweetener: Xylitab; sterol blend: commercially available blend of sterols and stanols, about 90% sterol content, mainly β-sitosterol, campesterol, stigmasterol and β-sitostanol, smaller amounts of other sterols, stanols and esters thereof, and further ingredients were mixed into a homogenous blend of particles in a standard mixer:

The resulting composition is seen in table 2.

**Table 2.**

| **Mixture for pressed tablets** | |
|---|---|
| **Ingredient** | **Wt%** |
| Chewing gum granules | 38.06 |
| High intensity sweeteners | 0.28 |
| Sterol blend | 11.69 |
| Xylitab | 46.57 |
| Peppermint blend | 2.5 |
| Menthol | 0.9 |

Before pressing, the mixtures passed a standard horizontal vibration sieve removing particles larger than 2.6 mm. The mixture was lead to a standard tablet pressing machine comprising dosing apparatus (P 3200 C, available from Fette GmbH, Germany) and pressed into compressed tablets.

Optionally a precompression step may be used prior to the compression step.

The sterols are relatively evenly distributed in the tablets of this example. The weight of the individual chewing gum tablets was about 2 g and comprised an amount of sterol compound of about 210 mg which may have desired physiological effects like lowering cholesterol levels in humans.

### Example 4

### Compressing the compositions into double-layer tablets with sterols in the gum base-containing layer

The resulting composition from Example 3 was used as a first gum base-containing layer in a double layer tablet in which the second gum base-free layer comprised
99% sorbitol
0.3% high-intensity sweetener
0.6% peppermint flavor
0.1% menthol powder

The sterol blend was thus placed in the layer comprising gum base.

The gum base layer comprising sterols accounted for about 70% by weight of the final tablet.

The gum base-containing granules forming the first layer were led to the mould of a standard tablet pressing machine comprising dosing apparatus (P 3200 C, available from Fette GmbH, Germany). The mixture was then pre-compressed to give a pre-compressed first layer with thickness of approximately 5 mm.

The gum base-free layer was fed into the mould of the tableting machine onto the first pre-compressed layer as a homogenous mixture of the above-mentioned ingredients to form a second layer.

The individual compressed tablets had a weight of about 2 g, a thickness of about 8.5 mm and it was seen that the interlayer cohesion was excellent. The tablet comprised an amount of sterol compound of about 147 mg which may have desired physiological effects like lowering cholesterol levels in humans.

### Example 5

### Compressing the compositions into double-layer tablets with sterol in the gum base-free layer

Table 3 shows the composition of a first gum base-containing layer and a second gum base-free layer in a double layer tablet.

**Table 3**

| **Ingredient** | **Mixture for pressed tablets** |
|---|---|
| **GB-containing layer** | **Wt% of layer** |
| Chewing gum granules | 40.58 |
| High intensity sweeteners | 0.28 |
| Xylitab | 55.74 |
| Peppermint blend | 2.5 |
| Menthol | 0.9 |

| **GB-free layer** | **Wt% of layer** |
|---|---|
| Sorbitol powder | 74.0 |
| Sterol blend | 25.0 |
| High intensity sweeteners | 0.3 |
| Peppermint | 0.6 |
| Menthol powder | 0.1 |

The sterol blend was comprised in the gum base-free layer.

The gum base-containing granules forming the first layer were lead to the mould of a standard tablet pressing machine comprising dosing apparatus (P 3200 C, available from Fette GmbH, Germany). The mixture was then pre-compressed to give a pre-compressed first layer.

The gum base-free layer was fed into the mould of the tableting machine onto the first pre-compressed layer as a homogenous mixture of particles of the above-mentioned ingredients to form a second layer. The gum base-free layer accounted for about 35% by weight of the final tablet.

The first and second layers were then compressed to form the layered tablet. The individual compressed tablets had a weight of about 2g and the interlayer cohesion was excellent. The tablet comprised an amount of sterol compound of about 158 mg which may have desired physiological effects like lowering cholesterol levels in humans.

### Example 6

### Compressing the compositions into double-layer tablets with sterol in both the gum base-containing layer and in the gum base-free layer

The resulting composition from Example 3, table 2, was used as a first gum base-containing layer in a double layer tablet in which the second gum base-free layer comprised:
65.7% sorbitol
33.3% sterol blend
0.3% high-intensity sweetener
0.6% peppermint
0.1 % menthol powder

The sterol blend, was thus placed in both the layer comprising gum base and the gum base-free layer.

The gum base-containing granules forming the first layer were lead to the mould of a standard tablet pressing machine comprising dosing apparatus (P 3200 C, available from Fette GmbH, Germany). The mixture was then pre-compressed with a force of 15 kN to give a pre-compressed first layer with thickness of approximately 5.0 mm.

The gum base-free layer was fed into the mould of the tableting machine onto the first pre-compressed layer as a homogenous mixture of the above-mentioned ingredients to form a second layer. The gum base-free layer accounted for about 30% by weight of the final tablet.

The first and second layers were then compressed to form the layered tablet. The individual compressed tablets had a weight of about 2 g, the gum base-free layer comprising about 30% by weight of the compressed chewing gum tablet. The interlayer adhesion was excellent. In this example, the sterol content of the compressed chewing gum tablet was approximately 282 mg, approximately 147 mg in the gum base-containing layer and about 135 mg in the gum base-free layer. The Compressed chewing gum tablet of this example thus comprised an amount of sterol which may have desired physiological effects like lowering cholesterol levels in humans.

### Example 7

### Sterol release measurements

20 pieces of compressed chewing gum tablets of example 3 were chewed for 20 minutes by test personnel. After chewing, the sterol content of the chewed tablets was measured by Gas Chromatography.

The average sterol-content after chewing was 40% of the initial sterol-content of the chewing gum tablet. This shows that about 60% of sterols initially present in the compressed chewing gum tablet is released and thereby administered to the person chewing the tablet for at least 20 minutes.

### Example 8

### Sterol release measurements

20 pieces of compressed chewing gum tablets of example 4 were chewed for 20 minutes by test personnel. After chewing, the sterol content of the chewed tablets was measured by Gas Chromatography.

The average sterol-content after chewing was 43% of the initial sterol-content of the chewing gum tablet. This shows that about 57% of sterols initially present in the compressed chewing gum tablet is released and thereby administered to the person chewing the tablet for at least 20 minutes.

### Example 9

### Sterol release measurements

20 pieces of compressed chewing gum tablets of example 5 were chewed for 20 minutes by test personnel. After chewing, the sterol content of the chewed tablets was measured by Gas Chromatography.

The average sterol-content after chewing was 13% of the initial sterol-content of the chewing gum tablet. This shows that about 87% of sterols initially present in the compressed chewing gum tablet is released and thereby administered to the person chewing the tablet for at least 20 minutes.

### Example 10

### Sterol release measurements

20 pieces of compressed chewing gum tablets of example 6 were chewed for 20 minutes by test personnel. After chewing, the sterol content of the chewed tablets was measured by Gas Chromatography.

The average sterol-content after chewing was 22% of the initial sterol-content of the chewing gum tablet. This shows that about 78% of sterols initially present in the compressed chewing gum tablet is released and thereby administered to the person chewing the tablet for at least 20 minutes.

### Example 11

### Subjective chew and taste parameters

Compressed chewing gum tablets from different chewing gum compositions were tested with respect to subjective chew parameters such as disintegration feel and taste.

Compressed chewing gum tablets from different chewing gum compositions varying in amount of bulk sweetener are listed in table 4.

**Table 4.**

| | **Composition No.** | | | |
|---|---|---|---|---|
| | 1 | 2 | 3 | 4 |
| **Chewing gum ingredients** | **Amount by %weight** | | | |
| Bulk sweetener | 26.0 | 31.0 | 41.0 | 53.0 |
| High intensity sweetener | 0.3 | 0.3 | 0.3 | 0.3 |
| Sterol compound | 15 | 15 | 15 | 15 |
| Chewing gum granules | 55.3 | 50.3 | 40.3 | 28.3 |
| Flavor | 3.4 | 3.4 | 3.4 | 3.4 |

Table 5 lists the result of the subjective parameter assessment. The results indicate a correlation between the amount of bulk sweetener comprised in the chewing gum composition and the disintegration feel, chew and taste experience. It can be seen that a certain amount of bulk sweetener may be advantageous to achieve a chewing gum tablet with good overall performance.

A similar effect may be achieved by varying the amount of high intensity sweetener. The tendency is the same that larger amounts of high intensity sweetener tend to mask the disintegration of the tablet in the mouth.

**Table 5**

| Composition No | Disintegration feel | Taste | Overall evaluation of chewing gum tablet chewing feel and taste |
|---|---|---|---|
| 1 | Poor | Poor | Poor |
| 2 | Acceptable | Acceptable | Good |
| 3 | Good | Good | Good |
| 4 | Good | Excellent | Excellent |

The disintegration feel is a subjective measure of how it feels when the compressed chewing gum tablet disintegrates in the mouth. A poor disintegration feel indicates that the amount of particles released upon initially chewing the tablet creates an unpleasant feeling. The disintegration feel is affected by the taste. A good taste may mask a poorer disintegration feel.

### Example 12

### Evaluation

The separation of gum base and sterol compound inherent in the chewing gum of this invention has shown very advantageous in comprising high amount of sterols and exhibiting advantageous release of sterols.

Chewing gum tablets of the present invention demonstrated by the above examples comprise sterols in biologically interesting amounts. The sterols are added as a partly water soluble dry powder and are kept separate from the gum base during the whole production process. The sterol release measurements of examples 7 - 9 show that up to 220 mg (calculated from examples 6 and 9) of sterol may be released from one chewing gum tablet upon chewing the tablet for at least 20 minutes.

Hereby distinct advantages when compared to prior art chewing gums, wherein the chewing gums comprise sterols directly in the gum base or in a liquid centre fill may be achieved.

## Claims

1. Compressed chewing gum tablet comprising at least one chewing gum module, the chewing gum module including a chewing gum composition comprising chewing gum particles containing gum base,
wherein said chewing gum tablet comprises at least one sterol compound, and
wherein at least a part of said at least one sterol compound is located outside said chewing gum particles containing gum base.

2. Compressed chewing gum tablet according to claim 1, wherein said at least one sterol compound is applied in powder form.

3. Compressed chewing gum tablet according to claim 1 or 2, wherein said sterol compound comprises one or more substances selected from the group consisting of sterols, stanols, sterol esters, stanol esters and derivatives thereof.

4. Compressed chewing gum tablet according to any of the claim 1-3, wherein at least a part of said at least one sterol compound is located in chewing gum particles free of gum base.

5. Compressed chewing gum tablet according to any of the claim 1-4, wherein said at least one sterol compound is contained in particles of which the at least one sterol compound make up more than 20, preferably more than 40, more preferably more than 60, even more preferably more than 80, and most preferably more than 90 % by weight of the particles.

6. Compressed chewing gum tablet according to any of the claim 1-5, wherein said sterol compound is separately added to the chewing gum composition located outside the chewing gum particles containing gum base, comprised in chewing gum particles free of gum base and/or combinations thereof.

7. Compressed chewing gum tablet according to any of the claim 1-6, wherein said chewing gum particles containing gum base comprise a part of said sterol compound.

8. Compressed chewing gum tablet according to any of the claim 1-7, wherein said compressed chewing gum tablet consists of one module or comprises at least two modules.

9. Compressed chewing gum tablet according to any of the claim 1-8, wherein said compressed chewing gum tablet comprises at least one gum base-free module and at least one gum base-containing module.

10. Compressed chewing gum tablet according to any of the claim 1-9, wherein said at least one gum base-free module and/or said at least one gum base-containing module comprises said at least one sterol compound.

11. Compressed chewing gum tablet according to any of the claim 1-10, wherein said at least one sterol compound is compressible or is mixed with compressible materials prior to compression.

12. Compressed chewing gum tablet according to any of the claim 1-11, wherein at least a part of said chewing gum particles free of gum base consist of at least one sterol compound comprising substances selected from the group consisting of sterols, stanols, sterol esters, stanol esters and derivatives thereof.

13. Compressed chewing gum tablet according to any of the claim 1-12, wherein said sterol compound comprises sterol esters and stanol esters in an amount of below 10% by weight of the sterol compound.

14. Compressed chewing gum tablet according to any of the claim 1-13, wherein said compressed chewing gum tablet comprises said at least one sterol compound in an amount of above 5 mg, preferably above 10 mg, most preferably above 20 mg.

15. Compressed chewing gum tablet according to claim 1, wherein the chewing gum composition further comprises chewing gum particles free of gum base, and
wherein at least a part of said chewing gum particles free of gum base comprise at least a part of said at least one sterol compound.

16. Tablet according to claim 15 in combination with any of the claims 2-14.

17. Method for production of a chewing gum according to any of the claims 1-16, wherein said method comprises at least one compression step.

## Patentansprüche

1. Komprimierte Kaugummitablette, umfassend mindestens ein Kaugummimodul, wobei das Kaugummimodul eine Kaugummi-Zusammensetzung einschließt, die Gummibasis enthaltende Kaugummiteilchen umfasst,
wobei die Kaugummitablette mindestens eine Sterol-Verbindung umfasst und wobei mindestens ein Teil der mindestens einen Sterol-Verbindung außerhalb der Gummibasis enthaltenden Kaugummiteilchen angeordnet ist.

2. Komprimierte Kaugummitablette nach Anspruch 1, bei der die mindestens eine Sterol-Verbindung in Pulverform angewendet ist.

3. Komprimierte Kaugummitablette nach Anspruch 1 oder 2, bei der die Sterol-Verbindung eine oder mehrere Substanzen umfasst, die ausgewählt sind aus der Gruppe bestehend aus Sterolen, Stanolen, Sterolestern, Stanolestern und deren Derivaten.

4. Komprimierte Kaugummitablette nach irgendeinem der Ansprüche 1 - 3, bei der mindestens ein Teil der mindestens einen Sterol-Verbindung in Kaugummiteilchen angeordnet ist, die frei von Gummibasis sind.

5. Komprimierte Kaugummitablette nach irgendeinem der Ansprüche 1 - 4, bei der die mindestens eine Sterol-Verbindung in Teilchen enthalten ist, bei denen die mindestens eine Sterol-Verbindung mehr als 20, bevorzugt mehr als 40, mehr bevorzugt mehr als 60, noch mehr bevorzugt mehr als 80, und am meisten bevorzugt mehr als 90 Gew.-% der Teilchen ausmacht.

6. Komprimierte Kaugummitablette nach irgendeinem der Ansprüche 1 - 5, bei der die Sterol-Verbindung getrennt zu der Kaugummi-Zusammensetzung gegeben wird, die außerhalb der Gummibasis enthaltenden Kaugummiteilchen angeordnet ist, in Kaugummiteilchen umfasst ist, die frei von Gummibasis sind, und/oder Kombinationen davon.

7. Komprimierte Kaugummitablette nach irgendeinem der Ansprüche 1 - 6, bei der die Gummibasis enthaltenden Kaugummiteilchen einen Teil der Sterol-Verbindung umfassen.

8. Komprimierte Kaugummitablette nach irgendeinem der Ansprüche 1 - 7, bei der die komprimierte Kaugummitablette aus einem Modul besteht oder mindestens zwei Module umfasst.

9. Komprimierte Kaugummitablette nach irgendeinem der Ansprüche 1 - 8, bei der die komprimierte Kaugummitablette mindestens ein Gummibasis-freies Modul und mindestens ein Gummibasis enthaltendes Modul umfasst.

10. Komprimierte Kaugummitablette nach irgendeinem der Ansprüche 1 - 9, bei der das mindestens eine Gummibasis-freie Modul und/oder das mindestens eine Gummibasis enthaltende Modul die mindestens eine Sterol-Verbindung umfasst.

11. Komprimierte Kaugummitablette nach irgendeinem der Ansprüche 1 - 10, bei der die mindestens eine Sterol-Verbindung komprimierbar ist oder vor der Kompression mit komprimierbaren Materialien gemischt wird.

12. Komprimierte Kaugummitablette nach irgendeinem der Ansprüche 1 - 11, bei der mindestens ein Teil der Gummibasis-freien Kaugummiteilchen aus mindestens einer Sterol-Verbindung besteht, die Substanzen umfasst, welche ausgewählt sind aus der Gruppe bestehend aus Sterolen, Stanolen, Sterolestern, Stanolestern und deren Derivaten.

13. Komprimierte Kaugummitablette nach irgendeinem der Ansprüche 1 - 12, bei der die Sterol-Verbindung Sterolester und Stanolester in einer Menge unterhalb von 10 Gew.-% der Sterol-Verbindung umfasst.

14. Komprimierte Kaugummitablette nach irgendeinem der Ansprüche 1 - 13, bei der die komprimierte Kaugummitablette die mindestens eine Sterol-Verbindung in einer Menge über 5 mg, vorzugsweise über 10 mg, am bevorzugtesten über 20 mg umfasst.

15. Komprimierte Kaugummitablette nach Anspruch 1, bei der die Kaugummi-Zusammensetzung weiter Kaugummiteilchen umfasst, die frei von Gummibasis sind, und
bei der mindestens ein Teil der Kaugummiteilchen, die frei von Gummibasis sind, mindestens einen Teil der mindestens einen Sterol-Verbindung umfasst.

16. Tablette nach Anspruch 15 in Kombination mit irgendeinem der Ansprüche 2 bis 14.

17. Verfahren zur Herstellung eines Kaugummis nach irgendeinem der Ansprüche 1 - 16, wobei das Verfahren mindestens einen Kompressionsschritt umfasst.

## Revendications

1. Tablette de gomme à mâcher comprimée comprenant au moins un module de gomme à mâcher, le module de gomme à mâcher comprenant une composition de gomme à mâcher comprenant des molécules de gomme à mâcher contenant une base de gomme,
ladite tablette de gomme à mâcher comprenant au moins un composé stérol, et
au moins une partie dudit au moins un composé stérol étant située à l'extérieur desdites particules de gomme à mâcher contenant une base de gomme.

2. Tablette de gomme à mâcher comprimée selon la revendication 1, dans laquelle ledit au moins un composé stérol est appliqué sous forme de poudre.

3. Tablette de gomme à mâcher comprimée selon la revendication 1 ou 2, dans laquelle ledit composé stérol comprend une ou plusieurs substances choisies dans le groupe constitué de stérols, stanols, esters de stérol, esters de stanol, et des dérivés de ceux-ci.

4. Tablette de gomme à mâcher comprimée selon l'une quelconque des revendications 1 à 3, dans laquelle au moins une partie dudit au moins un composé stérol est située dans des particules de gomme à mâcher exemptes de base de gomme.

5. Tablette de gomme à mâcher comprimée selon l'une quelconque des revendications 1 à 4, dans laquelle ledit au moins un composé stérol est contenu dans des particules dont l'au moins un composé stérol représente plus de 20, de préférence plus de 40, plus préférablement plus de 60, encore plus préférablement plus de 80, et de manière préférée entre toutes plus de 90 % en poids des particules.

6. Tablette de gomme à mâcher comprimée selon l'une quelconque des revendications 1 à 5, dans laquelle ledit composé stérol est ajouté séparément à la composition de gomme à mâcher située à l'extérieur des particules de gomme à mâcher contenant de la base de gomme, compris dans les particules de gomme à mâcher exemptes de base de gomme et/ou des combinaisons de ceux-ci.

7. Tablette de gomme à mâcher comprimée selon l'une quelconque des revendications 1 à 6, dans laquelle lesdites particules de gomme à mâcher contenant de la base de gomme comprennent une partie dudit composé stérol.

8. Tablette de gomme à mâcher comprimée selon l'une quelconque des revendications 1 à 7, dans laquelle ladite tablette de gomme à mâcher comprimée est constituée d'un module ou comprend au moins deux modules.

9. Tablette de gomme à mâcher comprimée selon l'une quelconque des revendications 1 à 8, dans laquelle ladite tablette de gomme à mâcher comprimée comprend au moins un module sans base de gomme et au moins un module contenant de la base de gomme.

10. Tablette de gomme à mâcher comprimée selon l'une quelconque des revendications 1 à 9, dans laquelle ledit au moins un module sans base de gomme et/ou ledit au moins un module contenant de la base de gomme comprend ledit au moins un composé stérol.

11. Tablette de gomme à mâcher comprimée selon l'une quelconque des revendications 1 à 10, dans laquelle ledit au moins un composé stérol est compressible ou est mélangé avec des matériaux compressibles avant compression.

12. Tablette de gomme à mâcher comprimée selon l'une quelconque des revendications 1 à 11, dans laquelle au moins une partie desdites particules de gomme à mâcher sans base de gomme sont constituées d'au moins un composé stérol comprenant des substances choisies dans le groupe constitué de stérols, de stanols, d'esters de stérol, d'esters de stanol et des dérivés de ceux-ci.

13. Tablette de gomme à mâcher comprimée selon l'une quelconque des revendications 1 à 12, dans laquelle ledit composé stérol comprend des esters de stérol et des esters de stanol en une quantité inférieure à 10 % en poids du composé stérol.

14. Tablette de gomme à mâcher comprimée selon l'une quelconque des revendications 1 à 13, dans laquelle ladite tablette de gomme à mâcher comprimée comprend ledit au moins un composé stérol en une quantité supérieure à 5 mg, de préférence supérieure à 10 mg, prédéterminé supérieure à 20 mg.

15. Tablette de gomme à mâcher comprimée selon la revendication 1, dans laquelle la composition de gomme à mâcher comprend en outre des particules de gomme à mâcher exemptes de base de gomme, et
dans laquelle au moins une partie desdites particules de gomme à mâcher exemptes de base de gomme comprennent au moins une partie dudit au moins un composé stérol.

16. Tablette selon la revendication 15 en combinaison avec l'une quelconque des revendications 2 à 14.

17. Procédé pour la production d'une gomme à mâcher selon l'une quelconque des revendications 1 à 16, dans lequel ledit procédé comprend au moins une étape de compression.
